# NEW EUROPEAN PATENT SPECIFICATION

(11) **EP 1 037 968 B2**
(45) Date of publication and mention of the opposition decision: **05.02.2014**
(45) Mention of the grant of the patent: 04.10.2006
(21) Application number: 98964823.3
(22) Date of filing: 18.12.1998
(51) Int. Cl.: C12N 9/98

(54) **MATRIX GRANULE**
IN EINEM WIRBELSCHICHTBETT HERGESTELLTES MATRIXGRANULAT
GRANULE A MATRICE

(30) Priority: 20.12.1997 US 995457; 27.10.1998 US 105874 P
(43) Date of publication of application: 27.09.2000
(73) Proprietor: GENENCOR INTERNATIONAL, INC., Palo Alto, California 94304 (US)
(72) Inventor: BECKER, Nathaniel, T., Palo Alto, CA 94304-1013 (US); CHRISTENSEN, Robert, I., Jr., Pinole, CA 94564 (US); GREEN, Thomas, S., Montara, CA 94307 (US)
(74) Representative: HOFFMANN EITLE
(86) International application number: PCT/US1998/027119
(87) International publication number: WO 1999/032613

(56) References cited:
- EP-A- 0 272 923
- EP-A- 0 304 332
- EP-A- 0 336 231
- EP-A- 0 351 162
- EP-A- 0 501 375
- WO-A-97/23606
- US-A- 5 739 091
- US-A- 5 814 501
- GAERTNER, A. L. ET AL: "Development of low dust enzyme detergent granules with high storage stability" PROC. INT. SYMP. CONTROLLED RELEASE BIOACT. MATER. (1998), 25TH, 289-290 CODEN: PCRMEY;ISSN: 1022-0178, XP002102189

## Description

### Background of the Invention

Proteins such as pharmaceutically important proteins like hormones and industrially important proteins like enzymes are becoming more widely used. Enzymes are used in several industries including, for example, the starch industry, the dairy industry, and the detergent industry. It is well known in the detergent industry that the use of enzymes, particularly proteolytic enzymes, has created industrial hygiene concerns for detergent factory workers, particularly due to the health risks associated with dustiness of the available enzymes.

Since the introduction of enzymes into the detergent business, many developments in the granulation and coating of enzymes have been offered by the industry.

U.S. Patent 4,106,991 describes an improved formulation of enzyme granules by including within the composition undergoing granulation, finely divided cellulose fibers in an amount of 2-40% w/w based on the dry weight of the whole composition. In addition, this patent describes that waxy substances can be used to coat the particles of the granulate.

U.S. Patent 4,689,297 describes enzyme containing particles which comprise a particulate, water dispersible core which is 150 - 2,000 microns in its longest dimension, a uniform layer of enzyme around the core particle which amounts to 10%-35% by weight of the weight of the core particle, and a layer of macro-molecular, film-forming, water soluble or dispersible coating agent uniformly surrounding the enzyme layer wherein the combination of enzyme and coating agent is from 25-55% of the weight of the core particle. The core material described in this patent includes clay, a sugar crystal enclosed in layers of corn starch which is coated with a layer of dextrin, agglomerated potato starch, particulate salt, agglomerated trisodium citrate, pan crystallized NaCl flakes, bentonite granules or prills, granules containing bentonite, kaolin and diatomaceous earth or sodium citrate crystals. The film forming material may be a fatty acid ester, an alkoxylated alcohol, a polyvinyl alcohol or an ethoxylated alkylphenol.

U.S. Patent 4,740,469 describes an enzyme granular composition consisting essentially of from 1-35% by weight of an enzyme and from 0.5-30% by weight of a synthetic fibrous material having an average length of from 100-500 micron and a fineness in the range of from 0.05-0.7 denier, with the balance being an extender or filler. The granular composition may further comprise a molten waxy material, such as polyethylene glycol, and optionally a colorant such as titanium dioxide.

U.S. Patent 5,324,649 describes enzyme-containing granules having a core, an enzyme layer and an outer coating layer. The enzyme layer and, optionally, the core and outer coating layer contain a vinyl polymer.

WO 91/09941 describes an enzyme containing preparation whereby at least 50% of the enzymatic activity is present in the preparation as enzyme crystals. The preparation can be either a slurry or a granulate.

WO 97/12958 discloses a microgranular enzyme composition. The granules are made by fluid-bed agglomeration which results in granules with numerous carrier or seed particles coated with enzyme and bound together by a binder.

Two of the methods known for preparing granulated enzymes in fluid-bed coaters include fluid-bed agglomeration and fluid-bed spray-coating. In fluid-bed agglomeration, one or more enzymes and a binder are sprayed on to fine powdery carrier solids, which are built up in size by agglomerating together carrier particles. In these agglomerates, the binder and enzyme serve to bridge multiple carrier particles into granules of irregular size and shape. In fluid-bed spray-coating, enzyme can be layered onto uniform core particles together with an optional binder.

It would be desirable to produce enzyme granules with improved stability, particularly in bleach-containing detergents at high humidity and temperature. Current fluid-bed spray-coated enzyme granules contain the enzyme in a relatively thin layer near the surface of the granule. This geometry renders the enzyme more vulnerable to being chipped off of the granule in a concentrated layer during handling and conveying operations, increasing the likelihood and levels of airborne enzyme aerosols in the working environment. This geometry also makes the enzyme more vulnerable to attack by penetrating moisture and inactivating substances.

However, even in light of these developments offered by the industry (as described above) there is a continuing need for low-dust enzyme granules which have additional beneficial characteristics. Additional beneficial characteristics needed in the enzyme granulation industry are low-residue granule formulations (where low residue is defined as a reduced tendency to leave noticeable undissolved residues on clothes or other material), and improved stability during storage in, for example, bleach-containing detergent formulas, for example, those containing peroxygen bleaches such as sodium perborate or sodium percarbonate. Accomplishing all these desired characteristics simultaneously is a particularly challenging task since, for example, many delayed release or low-dust agents such as fibrous cellulose or kaolin leave behind insoluble residues.

As such, there is a need for, for example, a detergent enzyme granule which is simultaneously non-dusting, stable when stored in detergents, and easy to manufacture in a controlled size distribution. Granules of a controlled size distribution are desirable in order to impart good flowability properties for handling and blending into detergents, and to resist segregation and settling once formulated into detergents. A controlled particle size distribution and uniform shape of particles are also important contributors to achieving a low dust granule.

Therefore, it is an object of the present invention to provide low-dust, low residue, high soluble enzyme granules having increased stability particularly in bleach-containing detergents. It is another object of the present invention to provide processes which afford the formation of such improved granules.

### Summary of the Invention

The present invention provides a layered granule having a single seed particle, with layers of said layered granule include a protein matrix layered over the seed particle and a barrier layer or coating layer. The protein matrix includes a protein mixed together with a combination of a sugar or sugar alcohol and a structuring agent. The barrier layer is layered over the protein matrix. Also, the coating can be applied over the protein matrix and/or the barrier layer. The structuring agent is a polysaccharide.

The present invention additionally provides a layered granule having a single seed particle, with layers of said layered granule include a enzyme matrix layered over the seed particle and a barrier layer or coating layer. The enzyme matrix includes a enzyme mixed together with a combination of a sugar or sugar alcohol and a structuring agent. The barrier layer is layered over the enzyme matrix. Also, the coating can be applied over the enzyme matrix and/or the barrier layer. The structuring agent is a polysaccharide.

### Detailed Description of the Invention

One embodiment of the invention is a layered granule having a single seed particle, with layers of said layered granule include a protein matrix layered over the seed particle and a barrier layer or coating layer. The protein matrix includes a protein mixed together with a combination of a sugar or sugar alcohol and a structuring agent. The barrier layer is layered over the protein matrix. Also, the coating can be applied over the protein matrix and/or the barrier layer. The structuring agent is a polysaccharide.

A further embodiment of the invention is a layered granule having a single seed particle, with layers of said layered granule include a enzyme matrix layered over the seed particle and a barrier layer or coating layer. The enzyme matrix includes a enzyme mixed together with a combination of a sugar or sugar alcohol and a structuring agent. The barrier layer is layered over the enzyme matrix. Also, the coating can be applied over the enzyme matrix and/or the barrier layer. The structuring agent is a polysaccharide.

The matrix is layered over a seed particle. There can be one or more layers between the seed particle and the matrix or the matrix and the barrier layer, for example, a coating such as polyvinyl alcohol (PVA).

Seed particles are inert particles upon which the enzyme matrix can be layered can be composed of inorganic salts, sugars, sugar alcohols, small organic molecules such as organic acids or salts, minerals such as clays or silicates or a combination of two or more of these. Suitable soluble ingredients for incorporation into seed particles include: sodium chloride, potassium chloride, ammonium sulfate, sodium sulfate, sodium sesquicarbonate, urea, citric acid, citrate, sorbitol, mannitol, oleate, sucrose, lactose and the like. Soluble ingredients can be combined with dispersible ingredients such as talc, kaolin or bentonite. Seed particles can be fabricated by a variety of granulation techniques including: crystallization, precipitation, pan-coating, fluid-bed coating, fluid-bed agglomeration, rotary atomization, extrusion, prilling, spheronization, drum granulation and high shear agglomeration. In the granules of the present invention the ratio of seed particles to granules is 1:1.

The "protein matrix", "enzyme matrix" or "matrix" is an admixture of one or more proteins such as an enzyme, a sugar or sugar alcohol and a structuring agent. The protein, sugar or sugar alcohol, and structuring agent can be mixed, for example, in solution or as a slurry. The protein can be applied from a solution or applied in slurry form as a suspension of crystals or precipitated protein. The matrix of the present invention comprises between about 20-80% of the weight of the granule.

By burying a protein within a matrix, the protein can be better protected from the twin dangers of attrition and activity loss. However it has not been possible previously to granulate enzymes in sugar or sugar alcohol matrices, since sugars and sugar alcohols exhibit "binder" characteristics, i.e. they are sticky and tend to plaster particles together (as happens intentionally in the case of granulation by agglomeration).

Also, to achieve a low dusting granular protein product, it is necessary to control the shape and size distribution of the granules. Uniform and reproducible size and shape also contribute to granule stability, since particle breakup and re-agglomeration would bring some protein near the granule surface.

Surprisingly, it has been found that by the addition of a structuring agent to the sugar matrix formula, protein can be applied uniformly to individual seed particles at rapid rates without agglomeration or attrition. The resulting particle size distribution can be precisely controlled, based on knowledge of the starting seed size distribution and the amount of solids to be added. The resulting particles are approximately spherical in shape, have high cohesive strength, and are resistant to attrition and penetration by moisture and inactivating substances.

Suitable sugars include sugars such as sucrose, glucose, fructose, raffinose, trehalose, lactose and maltose. Suitable sugar alcohols include sorbitol, mannitol and inositol. The amount of sugar or sugar alcohol in the matrix is preferably 0.1-90% by weight of the protein matrix. The sugar or sugar alcohol in the matrix can be sugar or sugar alcohol added to the protein or can be from the fermentation broth in which the protein is present.

The structuring agent is a polysaccharide. This class of compounds has the simultaneous desirable properties of high molecular weight and high water solubility. Without wishing to be bound by theory, it is believed that the high molecular weight of the structuring agent contributes two important properties which a sugar or sugar alcohol matrix alone would lack: (1) providing cohesion and strength to the particle, greatly reducing the tendency of the particle to dust; and (2) serving as a diffusion barrier to water and small molecules by virtue of forming a polymer network or "cage" throughout the matrix structure. This greatly improves tha stability of the granule.

The particular structuring agents chosen also typically have an anti-tack characteristic which is helpful in reducing the binder characteristic of the sugar or sugar alcohol, and allowing matrix layers to be built up-for example in fluid-bed coating-at rapid rates without agglomeration.

Sugars and sugar alcohols and structuring agents also have high water solubility or dispersibility. A matrix formula can be easily prepared which includes sugars or sugar alcohols, structuring agents, and enzymes as a solution or slurry with high total solids concentration. Total solution or slurry solids concentrations of 20-50% w/w or more can be formulated. These concentrated mixtures are highly desirable in that they can be formed into granules with a minimal need for evaporating water, an advantage in any granulation and drying process.

Preferred structuring agents include starch, modified starch, carrageenan, cellulose, modified cellulose, gum Arabic, guar gum, xanthan gum, and locust bean gum. Preferably, the structuring agent has low allergenicity. A combination of two or more structuring agents can be used in the granules of the present invention.

Proteins that are within the scope of the present invention include pharmaceutically important proteins such as hormones or other therapeutic proteins and industrially important proteins such as enzymes.

Any enzyme or combination of enzymes may be used in the present invention. Preferred enzymes include those enzymes capable of hydrolyzing substrates, e.g. stains. These enzymes are known as hydrolases which include, but are not limited to, proteases (bacterial, fungal, acid, neutral or alkaline), amylases (alpha or beta), lipases, cellulases and mixtures thereof. Particularly preferred enzymes are subtilisins and cellulases. Most preferred are subtilisins such as described in U.S. Patent 4,760,025, EP Patent 130 756 B1 and EP Patent Application WO 91/06637 and cellulases such as Multifect L250^{™} and Puradax^{™}, commercially available from Genencor International. Other enzymes that can be used in the present invention include oxidases, transferases, dehydratases, reductases, hemicellulases and isomerases.

The matrix of the granules of the present invention may further comprise one or more synthetic polymers or other excipients as known to those skilled in the art. Suitable synthetic polymers include polyethylene oxide, polyvinyl alcohol, polyvinyl pyrrolidone, polyethylene glycol and polyethylene oxide/polypropylene oxide.

The matrix may also further comprise plasticizers and anti-agglomeration agents. Suitable plasticizers useful in the present invention include polyols such as glycerol, propylene glycol, polyethylene glycol (PEG), urea, or other known plasticizers such as triethyl citrate, dibutyl or dimethyl phthalate or water. Suitable anti-agglomeration agents include fine insoluble or sparingly soluble materials such as talc, TiO₂, clays, amorphous silica, magnesium stearate, stearic acid and calcium carbonate.

The granules of the present invention can further comprise a barrier layer. A barrier layer is used to slow or prevent the diffusion of substances that can adversely affect the protein or enzyme into the matrix. The barrier layer is made up of a barrier material and is coated over the protein core. Suitable barrier materials include, for example, inorganic salts or organic acids or salts. The matrix without the protein can also be used as a barrier layer.

The granules of the present invention can also comprise one or more coating layers. For example, such coating layers may be one or more intermediate coating layers or such coating layers may be one or more outside coating layers or a combination thereof. Coating layers may serve any of a number of functions in a granule composition, depending on the end use of the enzyme granule. For example, coatings may render the enzyme resistant to oxidation by bleach, bring about the desirable rates of dissolution upon introduction of the granule into an aqueous medium, or provide a barrier against ambient moisture in order to enhance the storage stability of the enzyme and reduce the possibility of microbial growth within the granule.

Suitable coatings include water soluble or water dispersible film-forming polymers such as polyvinyl alcohol (PVA), polyvinyl pyrrolidone (PVP), cellulose derivatives such as methylcellulose, hydroxypropyl methylcellulose, hydroxycellulose, ethylcellulose, carboxymethyl cellulose, hydroxypropyl cellulose, polyethylene glycol, polyethylene oxide, gum arabic, xanthan, carrageenan, chitosan, latex polymers, and enteric coatings. Furthermore, coating agents may be used in conjunction with other active agents of the same or different categories.

Suitable PVAs for incorporation in the coating layer(s) of the granule include partially hydrolyzed, fully hydrolyzed and intermediately hydrolyzed PVAs having low to high degrees of viscosity. Preferably, the outer coating layer comprises partially hydrolyzed PVA having low viscosity. Other vinyl polymers which may be useful include polyvinyl acetate and polyvinyl pyrrolidone. Useful copolymers include, for example, PVA-methylmethacrylate copolymer and PVP-PVA copolymer.

The coating layers of the present invention may further comprise one or more of the following: plasticizers, extenders, lubricants, pigments, and optionally additional enzymes. Suitable plasticizers useful in the coating layers of the present invention are plasticizers including, for example, polyols such as sugars, sugar alcohols, or polyethylene glycols (PEGs), urea, glycol, propylene glycol or other known plasticizers such as triethyl citrate, dibutyl or dimethyl phthalate or water. Suitable pigments useful in the coating layers of the present invention include, but are not limited to, finely divided whiteners such as titanium dioxide or calcium carbonate or colored pigments and dyes or a combination thereof. Preferably such pigments are low residue pigments upon dissolution. Suitable extenders include sugars such as sucrose or starch hydrolysates such as maltodextrin and corn syrup solids, clays such as kaolin and bentonite and talc. Suitable lubricants include nonionic surfactants such as Neodol, tallow alcohols, fatty acids, fatty acid salts such as magnesium stearate and fatty acid esters.

Adjunct ingredients may be added to the enzyme granules of the present invention. Adjunct ingredients may include: metallic salts; solubilizers; activators; antioxidants; dyes; inhibitors; binders; fragrances; enzyme protecting agents/scavengers such as ammonium sulfate, ammonium citrate, urea, guanidine hydrochloride, guanidine carbonate, guanidine sulfamate, thiourea dioxide, monoethanolamine, diethanolamine, triethanolamine, amino acids such as glycine, sodium glutamate and the like, proteins such as bovine serum albumin, casein and the like etc.; surfactants including anionic surfactants, ampholytic surfactants, nonionic surfactants, cationic surfactants and long-chain fatty acid salts; builders; alkalis or inorganic electrolytes; bleaching agents; bluing agents and fluorescent dyes and whiteners; and caking inhibitors.

The granules described herein may be made by methods known to those skilled in the art of enzyme granulation, including pan-coating, fluid-bed coating, prilling, disc granulation, spray drying, extrusion, centrifugal extrusion, spheronization, drum granulation, high shear agglomeration, or combinations of these techniques.

The following examples are representative and not intended to be limiting. One skilled in the art could choose other enzymes, matrices, seed particles, methods and coating agents based on the teachings herein.

### Examples

### Example 1 (not according to the invention)

### Laboratory Fluid Bed Spray Coating of Alkaline Protease

1119 grams non-pareil particles (prepared by spraying a sucrose and corn starch colloidal mixture onto sucrose crystals and followed by spraying a final coating of PVA and corn starch and then sieved to between 20 and 50 mesh) were charged into a Vector FL1 fluid bed coater and fluidized. 159 grams of an aqueous solution containing 15% w/w Elvanol 51-05 (PVA marketed by Dow Chemical) was added to 1128 grams of an aqueous protease solution with 19.7% total dry solids and 8.4% w/w active protease. The protease/PVA solution was sprayed onto the non-pareils under the following conditions:

| | |
|---|---|
| Fluid feed rate | 18 g/min |
| Atomization pressure | 3.7 bar (54 psi) |
| Inlet air temperature set point | 100°C |
| Outlet air temperature range | 55 to 58°C |
| Inlet air rate | 48 m³/s (81 ft³/min) |

The coated particles were then coated with an aqueous solution containing 444 grams (40% w/w) of magnesium sulfate heptahydrate. This coating was applied under the following conditions:

| | |
|---|---|
| Fluid feed rate | 23 g/min |
| Atomization pressure | 3.7 bar (54 psi) |
| Inlet air temperature set point | 100°C |
| Outlet air temperature range | 55 to 58°C |
| Inlet air rate | 47 m³/s (80 ft³/min) |

The magnesium sulfate coated particles were then cosmetically coated with 2356 grams of an aqueous solution containing 146 grams (6.2% w/w) titanium dioxide, 118 grams (5% w/w) methylcellulose (Methocel A15-LV, Dow Chemical), 24 grams (1% w/w) of Neodol 23/6.5 (Shell Chemical Co.) and 39 grams (1.67% w/w) of polyethylene glycol at a molecular weight (MW) of 600. The cosmetic coating was applied under the following conditions:

| | |
|---|---|
| Fluid feed rate | 24 g/min |
| Atomization pressure | 3.7 bar (54 psi) |
| Inlet air temperature set point | 100°C |
| Outlet air temperature range | 51 to 58°C |
| Inlet air rate | 52 m³/s (88 ft³/min) |

A total of 1912 grams of enzyme granules were harvested as lot A. The overall mass balance for this experiment was 78%.

### Example 2

### Laboratory Fluid Bed Spray Coating of Alkaline Protease/Sucrose-Starch Matrix

404 grams of anhydrous sodium sulfate crystals sieved to between 50 and 70 mesh were charged into a Vector FL1 fluid bed coater and fluidized. 781 grams of an aqueous protease solution with 19.7% total dry solids and 8.4% w/w active protease was added to 1605 grams of an aqueous solution containing 670 grams of sucrose, 186 grams of common yellow dent starch and 74 grams of Ethylex 2015 (A. E. Staley , Decatur, Illinois) that had been fully hydrated by "cooking out" at 88°C (190°F) for 15 minutes. The ratio of enzyme solids to other solids in the combined solution was kept identical to Example 1, but the amounts were reduced to account for an extra step in this example. The combined solution was sprayed onto the sodium sulfate under the following conditions:

| | |
|---|---|
| Fluid feed rate | 27 g/min |
| Atomization pressure | 3.7 bar (54 psi) |
| Inlet air temperature set point | 100°C |
| Outlet air temperature range | 56 to 61 °C |
| Inlet air rate | 47 m³/s (80 ft³/min) |

The coated particles were then coated with an aqueous solution containing 444 grams (40% w/w) of magnesium sulfate heptahydrate. This coating was applied under the following conditions:

| | |
|---|---|
| Fluid feed rate | 27 g/min |
| Atomization pressure | 3.4 bar (50 psi) |
| Inlet air temperature set point | 100°C |
| Outlet air temperature range | 55 to 58°C |
| Inlet air rate | 46.5 m³/s (79 ft³/min) |

The magnesium sulfate coated particles were then cosmetically coated with 2356 grams of an aqueous solution containing 146 grams (6.2% w/w) titanium dioxide, 118 grams (5% w/w) methylcellulose, 24 grams (1% w/w) of Neodol 23/6.5 and 39 grams (1.67% w/w) of polyethylene glycol at a MW of 600. The cosmetic coating was applied under the following conditions:

| | |
|---|---|
| Fluid feed rate | 23 g/min |
| Atomization pressure | 3.9 bar (56 psi) |
| Inlet air temperature set point | 100°C |
| Outlet air temperature range | 53 to 58°C |
| Inlet air rate | 49 m³/s (83 ft³/min) |

A total of 2050 grams of enzyme granules were harvested as lot B. The overall mass balance for this experiment was 88.6%.

### Example 3

### Analysis of Lots

The granules of Examples 1 and 2 were analyzed to determine the amount of dust they generated and their stability in a three day stressed stability test. The methods for these procedures are as follows and the results are shown in Table 1.

### Accelerated Stability Test

The stability of many enzyme granules formulated into bleach-containing detergents is generally excellent, showing generally no more than about 10 to 20% loss in activity over 6 weeks storage at 30 to 37° C and 70% to 80% R.H. However, to aid in the development and screening of granular formulations, it is desirable to have an accelerated means of determining relative granule stability. The conditions of the accelerated stability test (AST) are far more severe than enzyme granules or detergents would ever encounter in realistic storage or transport. The AST is a "stress test" designed to discriminate differences between formulations which would otherwise not be evident for weeks or months.

In this test, a test detergent base was made from the following ingredients:

| | | |
|---|---|---|
| 72% | WFK-1 detergent base | (WFK, Forschunginstitut fuer Reinigungstechnologie e.V., Krefeld, Germany) |
| 25% | sodium perborate monohydrate | (Degussa Corp., Allendale Park, New Jersey.) |
| 3% | TAED bleach activator (= tetraacetylethylenediamine) | (Warwick International, Mostyn, UK) |

For each enzyme sample to be tested, three identical tubes were prepared by adding 1 gram of the test base and 30 mg of enzyme granules to a 15 ml conical tube and mixed by inverting the capped tube 5-8 times by hand. A hole was drilled in the tube cap with a 1/16 inch drill bit. One of the three tubes was assayed immediately and the other two were stored in a humidity chamber set at 50° C and 70%R.H. One of the two stored tubes was assayed after 1 day of storage; the second, after 3 days of storage. Storage stability was reported for Day 1 and Day 3 by dividing the remaining activity by the original activity at Day 0, expressed as a percentage.

The enzyme activity was determined by adding to each tube 30 ml of 0.25M MES pH 5.5 buffer containing 20 µl Catalase HP L5000 (Genencor International, Rochester, NY) and incubating for 40 minutes to inactivate the perborate. After this, the enzyme was assayed by adding 10 µl of the test tube mixture and 10 µl of sAAPF protease substrate to 980 µl of 0.1 M Tris pH 8.6, then incubating at 25°C over 3 minutes, and measuring the optical absorbance at 410 nm. The slope of the absorbance vs. time was then multiplied by the dilution factor and the known extinction coefficient for the specific protease to obtain an enzyme activity as concentration in mg/ml.

### Heubach attrition and elutriation dust tests.

Two commonly used methods for measuring enzyme granule dust are the Heubach attrition test and the elutriation test. These tests attempt to quantify the tendency of enzyme granules to generate airborne protein aerosols which might potentiate allergic reactions among workers in detergent plants. These tests are designed to reproduce certain mechanical actions typical of handling, conveying and blending operations used to mix enzyme granules into detergents at commercial scale.

In the elutriation test, 60 grams of enzyme granules were placed on a glass frit within a glass tube that was 175 cm high and 3.54 cm in diameter, and fluidized with a constant dry air stream at 0.8 meter/sec for 40 minutes.

In the Heubach attrition test, 13.5 g of granules were placed in a small, cylindrical chamber fitted with a rotating paddle and four steel balls; the granules were pushed around by the paddle and balls, while dry air percolated up through the chamber at 20 lpm for 20 minutes.

In both tests, dust stripped from the particles by the air was captured on a 15 cm tared glass fiber filter for subsequent weight measurement and activity determination by the sAAPF method described above. Enzyme dust for Heubach was reported as ng enzyme per gram of granules. Enzyme dust from elutriation was converted from activity to GU per 60g of granules, using enzyme-specific conversion factors.

**Table 1**

| Lot | Enzyme Core | Salt Layer | Coating Polymer | Bleach Det. Stability 3 days at 50°C | Heubach Total Dust (mg/pad) | Heubach Enz. Dust (ng/g) | Elutriation Dust (GU/60g) |
|---|---|---|---|---|---|---|---|
| A | Layered | MgSO₄ | MC | 34% | 1.8 | 2160 | 623 |
| B | Matrix | MgSO₄ | MC | 69% | 0.63 | 1058 | 130 |

### Example 4

### Pilot Scale Fluid Bed Spray Coating of Alkaline Protease/Sucrose-Starch Matrix

73.4 kg sucrose crystals sieved to between 35 and 50 mesh were charged into a modified Glatt WSG-120 fluid bed coater and fluidized. 174.67 kg of an aqueous protease solution with 19.98% total dry solids and 6.365% w/w active protease was added to 117 kg of an aqueous solution containing 36.25 kg of sucrose, 29 kg of common yellow dent starch and 7.25 kg of Ethylex 201 5 that had been fully hydrated by "cooking out" at 88°C (190°F) for 15 minutes. The combined solution was sprayed onto the sucrose under the following conditions:

| | |
|---|---|
| Fluid feed rate | 1.0 LPM |
| Atomization pressure | 5.2 bar (75 psi) |
| Inlet air temperature set point | NA |
| Product temperature set point | 70°C |
| Inlet air rate | 70 cubic meters/min |

The coated particles were then coated with an aqueous solution containing 75 kg (40.3% w/w) of magnesium sulfate heptahydrate. This coating was applied under the following conditions:

| | |
|---|---|
| Fluid feed rate | 2.3 LPM |
| Atomization pressure | 3.4 bar (50 psi) |
| Inlet air temperature set point | NA |
| Product temperature set point | 70°C |
| Inlet air rate | 70 cubic meters/min |

The magnesium sulfate coated particles were then cosmetically coated with 208.93 kg of an aqueous solution containing 12.97 kg (6.2% w/w) titanium dioxide, 10.59 kg (5% w/w) methylcellulose, 2.12 kg (1% w/w) of Neodol 23/6.5 and 3.57 kg (1.67% w/w) of polyethylene glycol at a MW of 600. The cosmetic coating was applied under the following conditions:

| | |
|---|---|
| Fluid feed rate | 1.1 LPM |
| Atomization pressure | 5.2 bar (75 psi) |
| Inlet air temperature set point | NA |
| Product temperature set point | 75°C |
| Inlet air rate | 70 cubic meters/min |

A total of 199.35 kg of enzyme granules were harvested as lot D. The overall mass balance for this experiment was 83.84%,

### Example 5

### Pilot Scale Fluid Bed Spray Coating of Alkaline Protease/Sucrose-Starch Matrix

### A.

65.75 kg sucrose crystals sieved to between 35 and 50 mesh were charged into a modified Glatt WSG-120 fluid bed coater and fluidized. 180.42 kg of an aqueous protease solution with 20.74% total dry solids and 6.71% w/w active protease was added to 145.13 kg of an aqueous solution containing 37.57 kg of sucrose, 29.94 kg of common yellow dent starch and 7.62 kg of Ethylex 2015 that had been fully hydrated by "cooking out" at 88°C (190°F) for 15 minutes. The combined solution was sprayed onto the sucrose under the following conditions:

| | |
|---|---|
| Fluid feed rate | 1.0 LPM |
| Atomization pressure | 5.2 bar (75 psi) |
| Inlet air temperature set point | NA |
| Product temperature set point | 70°C |
| Inlet air rate | 58 cubic meters/min |

### B.

The coated particles were then coated with an aqueous solution containing 86.95 kg (40.3% w/w) of magnesium sulfate heptahydrate. This coating was applied under the following conditions:

| | |
|---|---|
| Fluid feed rate | 1.7 LPM |
| Atomization pressure | 3.4 bar (50 psi) |
| Inlet air temperature set point | NA |
| Product temperature set point | 50°C |
| Inlet air rate | 58 cubic meters/min |

The magnesium sulfate coated particles were then cosmetically coated with 240.79 kg of an aqueous solution containing 16.97 kg (6.2% w/w) titanium dioxide, 6.84 kg (2.5% w/w) methylcellulose, 6.84 kg (2.5% w/w) of maltodextrin M150 (DE=15 from Grain Processing Corp., Muscatine, lowa), 2.74 kg (1% w/w) of Neodol 23/6.5 and 4.57 kg (1.67% w/w) of polyethylene glycol at a MW of 600. The cosmetic coating was applied under the following conditions:

| | |
|---|---|
| Fluid feed rate | 1.2 LPM |
| Atomization pressure | 5.2 bar (75 psi) |
| Inlet air temperature set point | NA |
| Product temperature set point | 60°C |
| Inlet air rate | 58 cubic meters/min |

A total of 199.35 kg of enzyme granules were harvested as lot E. The overall mass balance for this experiment was 97.13%,

### Example 6

### Pilot Scale Fluid Bed Spray Coating of Alkaline Protease/Sucrose-Starch Matrix

The enzyme cores were made according to section A of Example 5.

In the following three granules, the magnesium sulfate heptahydrate was applied as a 50% solution so as to constitute 15% by weight of the final granule weight. The conditions were as follows:

| | |
|---|---|
| Atomization pressure | 3.4 bar (50 psi) |
| Inlet air temperature set point | NA |
| Product temperature set point | 47-54°C |
| Inlet air rate | 58 cubic meters/min |

The coating polymers were applied as 15 % w/w solutions of soluble solids, batched in order to deliver the following coating compositions, given as weight percentages of the final granules in Table 2. The conditions were as follows:

| | |
|---|---|
| Atomization pressure | 3.4 bar (50 psi) |
| Inlet air temperature set point | NA |
| Product temperature set point | 46-55°C |
| Inlet air rate | 58 cubic meters/min |

**Table 2**

| Lot | MC (%) | MD (%) | Sucrose (%) | PEG (%) | Neodol (%) | TiO₂ (%) |
|---|---|---|---|---|---|---|
| F | 2.5 | 2.5 | | 1.7 | 1.0 | 5.0 |
| G | 1.5 | | 3.0 | 1.7 | 1.5 | 5.0 |
| H | 2.5 | 2.5 | | 1.7 | 1.0 | |

The granules were analyzed as described in Example 3 and the results are shown in Table 3.

**Table 3**

| Lot | Enzyme Core | Salt Layer | Coating Ingredients | Bleach Det. Stability 3 days, 50C | Heubach Total Dust (mg/pad) | Heubach Enz. Dust (ng/g) | Elutriatio n Dust (GU/60g) |
|---|---|---|---|---|---|---|---|
| F | Matrix | MgSO₄ | MC, MD, PEG Neodol, TiO₂ | 65% | 0.6 | 481 | 23 |
| G | Matrix | 4 | MC, sucrose, PEG Neodol, TiO₂ | 55% | 8.2 | 437 | 101 |
| H | Matrix | MgSO₄ + 5%TiO 2 | MC, MD, PEG Neodol | 73% | 0.5 | 370 | 34 |

### Example 7

Three large scale matrix granules were produced in a modified Glatt WSG 120 fluidized bed spray-coater. In Lot J, 50.5 kg of -35/+50 mesh sucrose seeds were charged into the coater and fluidized. A matrix carrier solution was prepared by cooking out 0.4 kg of Ethylex 2015 starch, as in the previous examples, and adding 46.7 kg sucrose and 23.4 kg dry yellow dent corn starch, with water added to give a final solution weight of 337.4 kg. The matrix carrier solution was combined with 243.2 kg of an aqueous protease solution containing 51.89 g/L GG36 subtilisin and 19% total solids, to form the enzyme matrix solution. The enzyme matrix solution was sprayed onto the sucrose seeds under the following conditions:

| | |
|---|---|
| Bed temperature: | 60°C |
| Fluidization air: | 28 m³/s at standard conditions (48 scfm) |
| Spray rate ramp: | 0.3 to 1.0 Ipm over 240 minutes |
| Atomization air: | 3.4-5.2 bar excess pressure (50-75 psig) over 240 minutes |

A solution of ammonium sulfate was prepared by dissolving 58.3 kg of ammonium sulfate in 135.9 kg water and this was sprayed over the matrix-coated seeds under the following conditions:

| | |
|---|---|
| Bed temperature: | 70°C |
| Fluidization air: | 28-34 m³/s at standard conditions (48-57 scfm) |
| Spray rate: | 1.5 Ipm |
| Atomization air: | 5.2 bar excess pressure (75 psig) |

Finally, a coating solution was prepared by dissolving or suspending 17.9 kg Elvanol 51-05 polyvinyl alcohol, 22.4 kg titanium dioxide, and 4.5 kg Neodol 23.5-6T nonionic surfactant in water to a net weight of 224.1 kg. This coating solution was applied under the following conditions:

| | |
|---|---|
| Bed temperature: | 72°C |
| Fluidization air: | 33 m³/s at standard conditions (56 scfm) |
| Spray rate: | 0.5-1.2 Ipm over 300 minutes |
| Atomization air: | 5.2 bar excess pressure (75 psig) |

After the coating was completed, 255.5 kg of granules were harvested from the coater and sieved to retain the -16/+50 mesh cut. The granule was assayed at 4.54 % w/w active subtilisin, and dust and stability measurements were conducted, reported in the table below.

Two additional batches of matrix granules, Lots K and L, were produced in the modified Glatt WSG 120 coater under essentially the same process conditions, but with the formulation changes noted in the table below. A layered granule was produced as described in Example 1.

Table 4 below summarizes the four formulations and reports both stability and dust for each sample.

**Table 4**

| PARAMETER | Layered Granule | Matrix Granule Lot J | Matrix Granule Lot K | Matrix Granule Lot L |
|---|---|---|---|---|
| Weights (kg) | | | | |
| Sucrose seeds | NA | 50.5 | 38.2 | 58.8 |
| Sucrose | NA | 46.7 | 17.8 | 24.1 |
| Dry starch | NA | 23.4 | 41.8 | 53.6 |
| Gelled starch | NA | 0.4 | 0 | 0 |
| Enzyme liquid | NA | 243 | 88 | 133 |
| Enzyme activity (g/L) | NA | 51. 9 | 49.9 | 67.1 |
| Salt | NA | 58.3 | 48.4 | 38.7 |
| TiO₂ | NA | 22.4 | 7.9 | 12.6 |
| PVA (Elvanol 51-05) | NA | 17.9 | 10.8 | 10.8 |
| Neodol 23.5-6T | NA | 4.5 | 2.7 | 2.5 |
| Ratios (% or % w/w) | | | | |
| Enzyme Payload | 2.00 | 4.54 | 2.70 | 3.35 |
| Dry starch: sucrose | NA | 0.50 | 2.34 | 2.22 |
| Gelled starch:sucrose | NA | 0.01 | 0 | 0 |
| Salt type | (NH₄)₂SO₄ | (NH₄)₂SO₄ | MgSO₄ | MgSO₄ |
| Salt level (% w/w) | 20 | 22 | 30 | 20 |
| PVA | 6.8 | 7.0 | 6. 7 | 5.4 |
| TiO₂ | 5.4 | 8.8 | 4.9 | 6.4 |
| Neodol | 1.4 | 1.7 | 1.7 | 1.3 |
| 3-Day Stability (%) | 29.8 | 95.2 | 67.9 | 79.9 |
| Heubach Dust | | | | |
| Total Dust (mg/pad) | | 0.4 | 0.4 | 0.4 |
| Enzyme Dust (ng/g) | | 56 | 174 | 78 |

### Example 8 (not according to the invention)

### Pilot Scale Fluid Bed Spray Coating of Amylase/Starch Matrix

26 kg sucrose crystals sieved to between 35 and 50 mesh were charged into Deseret 60 fluid bed coater and fluidizer. 15.3 kg of an aqueous amylase solution with 31% total dry solids and 12.5% w/w active amylase was added to 43.5 kg of an aqueous solution containing 23.5 kg of corn starch. The combined solution was sprayed onto the sucrose under the following conditions:

| | |
|---|---|
| Fluid feed rate | 0.8 kg/min |
| Atomization pressure | 5.2 bar (75 psi) |
| Inlet air temperature set point | NA |
| Product temperature set point | 45°C |
| Inlet air rate | 770 m³/s (1300 ft³/min) |

The coated particles were then coated with an aqueous solution containing 66.7 kg (40% w/w) of magnesium sulfate heptahydrate. This coating was applied under the following conditions:

| | |
|---|---|
| Fluid feed rate | 1.1 kg/min |
| Atomization pressure | 4.1 bar (60 psi) |
| Inlet air temperature set point | NA |
| Product temperature set point | 47°C |
| Inlet air rate | 1060 m³/s (1800 ft³/min) |

The magnesium sulfate coated particles were then cosmetically coated with 92.6 kg of an aqueous solution containing 7.1 kg (6.2% w/w) titanium dioxide, 2.9 kg (2.5% w/w) methylcellulose, 2.9 kg (2.5%) Purecote B790, 1.2kg (1.5% w/w) Neodol 23/6.5, and 2.0 kg (1.67% w/w) of polyethylene glycol at a MW of 600. The cosmetic coating was applied under the following conditions:

| | |
|---|---|
| Fluid feed rate | 0.5 kg/min |
| Atomization pressure | 5.2 bar (75 psi) |
| Inlet air temperature set point | NA |
| Product temperature set point | 47°C |
| Inlet air rate | 1060 m³/s (1800 ft³/min) |

### Example 9

### Pilot Scale Fluid Bed Spray Coating of Amylase/Sucrose-Starch Matrix

26 kg sucrose crystals sieved to between 35 and 50 mesh were charged into Deseret 60 fluid bed coater and fluidizer. 15.3 kg of an aqueous amylase solution with 31 % total dry solids and 12.5% w/w active amylase was added to 59.3 kg of an aqueous solution containing 7.8 kg of sucrose and 23.5 kg of corn starch. The combined solution was sprayed onto the sucrose under the following conditions:

| | |
|---|---|
| Fluid feed rate | 0.8 kg/min |
| Atomization pressure | 5.2 bar (75 psi) |
| Inlet air temperature set point | NA |
| Product temperature set point | 45°C |
| Inlet air rate | 770 m³/s (1300 ft³/min) |

The MgSO4 and cosmetic coating were run exactly as described above in Example 8.

### Example 10

In a modified Glatt WSG 120 fluidized bed spray coater, 47.37 kg sucrose crystals, sized at 30-50 mesh, were added and fluidized at 40-60 m3/min and 45 degrees C. An amylase enzyme suspension was prepared by slurrying 67.72 kg of common yellow dent corn starch in 105 kg of amylase UF concentrate (LAT) with an activity of 30,000 TAU/g or 85.7 mg/g amylase and which contained 24.2 mg/ml sugars carried forward from the fermentation and recovery processes. The enzyme suspension was coated onto the sucrose seeds under the following conditions (where a range is shown, the values are linearly increased over a ramp period):

| | |
|---|---|
| Ramp time: | 90 minutes |
| Fluid feed rate | 0.9-1.35 liter/min |
| Atomization pressure | 3.1-5.2 bar (45-75 psi) |
| Inlet air temperature | adjusted to maintain outlet air temperature |
| Outlet air temperature | 45°C |
| Fluidization air rate | 40-60 m³/min |

After the enzyme suspension was coated onto the sucrose crystals, 80 kg of a 50% solution of MgSO4 heptahydrate was sprayed onto the fluidized granules under the following conditions:

| | |
|---|---|
| Ramp time: | 30 minutes |
| Fluid feed rate | 1.12-2.15 liter/min |
| Atomization pressure | 4.1 bar (60 psi) |
| Inlet air temperature | adjusted to maintain outlet air temperature |
| Outlet air temperature | 45°C |
| Fluidization air rate | 60 m³/min |

Finally, a coating solution was prepared by adding 5.29 kg Methocel A-15 methycellulose (Dow Chemical), 12.71 kg titanium dioxide (DuPont), 5.29 kg Pure Cote B-790 modified starch (Grain Processing Corp.), 2.12 kg Neodol 23-6.5T (Shell) and 3.54 kg polyethyelene glycol, molecular weight 600 (Union Carbide) to 174.91 kg of heated water and cooling to about 20 degrees C to fully dissolve the polymers. The coating solution is applied under the following conditions:

| | |
|---|---|
| Ramp time: | 60 minutes |
| Fluid feed rate | 0.75-1.3 liter/min |
| Atomization pressure | 5.2 bar (75 psi) |
| Inlet air temperature | adjusted to maintain outlet air temperature |
| Outlet air temperature | 45°C |
| Fluidization air rate | 60 m³/min |

The resulting 180 kg of coated amylase matrix granules were harvested from the coater, with an enzyme yield of 85%.

## Claims

1. A layered granule having a single seed particle, layers of the layered granule comprising:
a) a protein matrix layered over the seed particle wherein said matrix comprises a mixture of a protein and a combination of a sugar or sugar alcohol and a polysaccharide structuring agent; and
b) a barrier layer coated over the protein matrix or a coating layer.

## Patentansprüche

1. Geschichtetes Granulat mit einem einzelnen Keimpartikel, wobei Schichten des geschichteten Granulats folgendes umfassen:
(a) eine über das Keimpartikel geschichtete Proteinmatrix, wobei die Matrix eine Mischung aus einem Protein und einer Kombination eines Zuckers oder Zuckeralkohols und eines Polysaccharid-Strukturierungsmittels umfasst; und
(b) eine Sperrschicht, die über die Proteinmatrix geschichtet ist, oder eine Überzugsschicht.

## Revendications

1. Granule en couches ayant une particule germe unique, les couches du granule en couches comprenant :
a) une matrice protéique disposée en plusieurs couches sur la particule germe, dans laquelle ladite matrice comprend un mélange d'une protéine et d'une combinaison d'un sucre ou d'un alcool de sucre et d'un agent structurant tel qu'un polysaccharide ; et
b) une couche barrière déposée sur la matrice protéique ou une couche d'enrobage.
